# EUROPÄISCHE PATENTSCHRIFT

(11) **EP 1 387 666 B1**
(45) Veröffentlichungstag und Bekanntmachung des Hinweises auf die Patenterteilung: **23.01.2008**
(21) Anmeldenummer: 02735289.7
(22) Anmeldetag: 24.04.2002
(51) Int. Cl.: A61K 8/03, A61K 8/46, A61K 8/49, A61K 8/92, A61Q 5/02, A61Q 19/10

(54) **ZWEI-PHASEN-REINIGUNGSZUBEREITUNGEN MIT HOHEM ÖLGEHALT**
TWO-PHASE CLEANSING AGENTS HAVING A HIGH OIL CONTENT
PREPARATIONS NETTOYANTES A DEUX PHASES A FORTE TENEUR EN HUILE

(30) Priorität: 30.04.2001 DE 10120825
(43) Veröffentlichungstag der Anmeldung: 11.02.2004
(73) Patentinhaber: Beiersdorf AG, 20245 Hamburg (DE)
(72) Erfinder: RUPPERT, Stephan, 20259 Hamburg (DE)
(86) Internationale Anmeldenummer: PCT/EP2002/004472
(87) Internationale Veröffentlichungsnummer: WO 2002/087539

(56) Entgegenhaltungen:
- EP-A- 0 210 774
- EP-A- 0 867 176
- DE-A- 19 835 239
- US-A- 3 533 955
- US-A- 3 964 500

## Beschreibung

Die vorliegende Erfindung betrifft wasserfreie kosmetische oder dermatologische Reinigungszubereitungen, welche zwei Phasen aufweisen und sich durch einen hohen Ölghalt auszeichnen.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z. B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden. Es hat daher nicht an Versuchen gefehlt, geeignete Reinigungszubereitungen zur gleichzeitigen Regeneration der Haut zu finden.

Bekannte Mittel zur Reinigung und gleichzeitigen Pflege der Haut sind z. B. Wannenbadzubereitungen, insbesondere Ölbad- oder Ölcrèmebadzubereitungen. Sie bieten ein Maximum an Pflege und Rückfettung während des Badens und sind üblicherweise, da Lipide den Schaum sehr stark herabsetzen, praktisch nicht oder allenfalls schwach schäumend.

Je nach Aufbau des Produktes können die Pflegeöle im Badewasser milchig dispergiert oder als dünner Film auf der Wasseroberfläche angereichert sein.

Der Stand der Technik kennt Ölbadzubereitungen verschiedener Art, wobei die Eigenschaften der Fett- oder Ölphase durch Zugabe von oberflächenaktiven Substanzen variiert werden können. Dabei können je nach Art und Menge der gewählten Bestandteile Zubereitungen formuliert werden, die auf der Badewasseroberfläche entweder spreitende Ölfilme, Öl-in-Wasser-Systeme oder auch Totalsolubilisate ergeben. Ölbäder werden im allgemeinen zur Behandlung extrem trockener Hautzustände eingesetzt. Sie enthalten, sofern sie als Badezusätze eingesetzt werden, in der Regel auch Emulgatoren, die eine Verteilung des Öls im Badewasser erlauben.

Eine relativ neue Entwicklung, die vom Verbraucher sehr positiv angenommen wird, sind Zwei-Phasen-Ölbader, welche vor Gebrauch durch Schütteln vermischt werden können. Nach dem Gebrauch entmischen sich die Produkte innerhalb weniger Stunden wieder. Durch unterschiedliche Färbung der Phasen wird dem Verbraucher das Vorliegen zweier getrennter Phasen angezeigt. Eine ausreichende Durchmischung ist erreicht, wenn das Produkt homogen gefärbt ist. Es handelt sich dabei entweder um Produkte mit einer wässrigen und einer Ölphase bzw. Emulsionsphase, deren kurzzeitige Emulgierung durch schäumende Tenside erreicht wird oder um Produkte aus zwei nicht mischbaren Ölen, die kein schäumendes Tensid enthalten.

Es war Aufgabe der vorliegenden Erfindung, den Stand der Technik um Zwei-Phasen-Ölbader zu bereichern, welche sich dabei durch sehr gute Schaumleistung und kosmetische Leistungen, wie Pflegeeffekt, Reinigungswirkung und dergleichen auszeichnen.

Gegenstand der vorliegenden Erfindung sind daher kosmetische oder dermatologische Reinigungszubereitungen, welche in mindestens zwei Phasen vorliegen, enthaltend
(a) mindestens ein waschaktives Tensid, gewählt aus der Gruppe der Trialkyl- und/oder Trialkanolaminsalze der Fettalkoholsulfate und Fettalkoholethersulfate,
(b) einen Gehalt von 20 bis 70 Gew.-%, bezogen auf das Gesamtgewicht der Zubereitungen, einer oder mehrerer Ölkomponenten, gewählt aus der Gruppe der unpolaren Lipide mit einer Polarität ≥ 30 mN/m und
(c) mindestens einem Polysorbat.

Die erfindungsgemäßen Zubereitungen sind wasserfrei. Hierzu sind im Sinne der vorliegenden Erfindung auch solche Zubereitungen zu rechnen, denen kein Wasser zugesetzt wird, welche aber produktionsbedingt - beispielweise durch Verwendung bestimmter wasserhaltiger Rohstoffe - geringe Mengen Wasser enthalten können.

Besonders bevorzugt sind erfindungsgemäße Zubereitungen, welche neben den obengenannten Bestandteilen Glycerin (Propantriol) enthalten. Glycerin ist eine farblose, klare, hochviskose, geruchlose, süß schmeckende, hygroskopische, praktisch ungiftige Flüssigkeit, die mit Wasser und Alkohol in jedem Verhältnis mischbar ist. Glycerin ist in verschiedenen Reinheiten erhältlich. Es ist vorteilhaft im Sinne der vorliegenden Erfindung, wenn das Glycerin möglichst rein ist, also beispielsweise "chemisch rein" nach DAB, insbesondere auch "reinst" nach DAB.

Die erfindungsgemäßen Zubereitungen haben eine sehr gute Schaumentwicklung, starke Reinigungskraft und entfalten eine hohe Hautpflegewirkung. Insbesondere wirken die erfindungsgemäßen Zubereitungen hautglättend, vermindern das Trockenheitsgefühl der Haut und machen die Haut geschmeidig.

Vorteilhaft weisen die erfindungsgemäß günstig zu verwendenden Fettalkoholsulfate bzw. Fettalkoholethersulfate folgende Struktur auf:

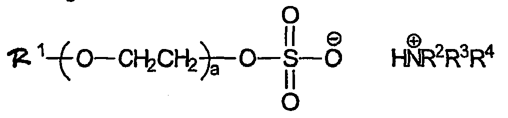

Dabei kann a Werte von 0 bis 10, vorteilhaft 1 bis 5 annehmen. R¹ wird gewählt aus der Gruppe der verzweigten und unverzweigten Alkylgruppen mit 6 bis 24 Kohlenstoffatomen. R², R³ und R⁴ werden unabhängig voneinander gewählt aus der Gruppe der verzweigten und unverzweigten Alkyl- und Hydroxyalkylreste mit 1 bis 24 Kohlenstoffatomen.

Herstellungsbedingt können die verwendeten Tenside Restmengen an physiologisch unbedenklichen, nicht umgesetzen Edukten enthalten, beispielsweise 1,2-Propylenglykol.

Bevorzugtes Fettalkoholethersulfat ist TIPA-Laurethsulfat.

Die Ölphase der erfindungsgemäßen Zubereitungen wird vorteilhaft gewählt aus der Gruppe der unpolaren Lipide mit einer Polarität ≥ 30 mN/m. Besonders vorteilhafte unpolare Lipide im Sinne der vorliegenden Erfindung sind die im folgenden aufgelisteten.

| **Hersteller** | **Handelsname** | **INCI-Name** | **Polarität mN/m** |
|---|---|---|---|
| Total SA | Ecolane 130 | Cycloparaffin | 49,1 |
| Neste PAO N.V. (Lief. Hansen & Rosenthal) | Nexbase 2006 FG | Polydecene | 46.7 |
| Chemische Fabrik Lehrte | Polysynlane | Hydrogenated Polyisobutene | 44.7 |
| Wacker | Wacker Silikonöl AK 50 | Polydimethylsiloxan | 46,5 |
| EC Erdölchemie (Lieferant Bayer AG) | Solvent ICH | Isohexadecane | 43.8 |
| DEA Mineralöl (Lief. Hansen & Rosenthal) Tudapetrol | Pionier 2076 | Mineral Oil | 43.7 |
| DEA Mineralöl (Lief. Hansen & Rosenthal) Tudapetrol | Pionier 6301 | Mineral Oil | 43.7 |
| Wacker | Wacker Silikonöl AK 35 | Polydimethylsiloxan | 42,4 |
| EC Erdölchemie GmbH | Isoeikosan | Isoeikosan | 41.9 |
| Wacker | Wacker Silikonöl AK 20 | Polydimethylsiloxan | 40,9 |
| Condea Chemie | Isofol 1212 Carbonat | | 40,3 |
| Gattefossé | Softcutol O | Ethoxydiglycol Oleate | 40,5 |
| Creaderm | Lipodermanol OL | Decyl Olivate | 40,3 |
| Henkel | Cetiol S | Dioctylcyclohexane | 39,0 |
| DEA Mineralöl (Lief. Hansen & Rosenthal) Tudapetrol | Pionier 2071 | Mineral Oil | 38.3 |
| WITCO BV | Hydrobrite 1000 PO | Paraffinum Liquidum | 37,6 |
| Goldschmidt | Tegosoft HP | Isocetyl Palmitate | 36,2 |
| Condea Chemie | Isofol Ester 1693 | | 33,5 |
| Condea Chemie | Isofol Ester 1260 | | 33,0 |
| Dow Coming | Dow Coming Fluid 245 | Cyclopentasiloxan | 32,3 |
| Unichema | Prisorine 2036 | Octyl Isostearate | 31.6 |
| Henkel Cognis | Cetiol CC | Dicaprylyl Carbonate | 31,7 |
| ALZO (ROVI) | Dermol 99 | Trimethylhexyl Isononanoate | 31,1 |
| ALZO (ROVI) | Dermol 89 | 2- Ethylhexyl Isononanoate | 31,0 |
| Unichema | Estol 1540 EHC | Octyl Cocoate | 30.0 |

Es ist insbesondere bevorzugt, wenn die Ölphase der erfindungsgemäßen Zubereitungen zum mehr als 50% aus Mineralöl besteht.

Vorteilhaft enthalten die Ölbäder im Sinne der vorliegenden Erfindung ferner auch Türkischrotöle.

Türkischrotöl (auch: Sulforicinat, Sulforicinoleat oder turkey red oil, CTFA-Bezeichnung: Sulfated Castor Oil, CAS-Nr.8002-33-3) ist die Bezeichnung für klare, gelbbraune, flüssige Gemenge aus verschiedenen Verbindungen (wie z. B. Ricinusöl, Ricinusölfettsäuren, Schwefelsäureester der Ricinusölfettsäuren, Dioxystearinsäure und deren Schwefelsäureester, Lactone usw.). Türkischrotöl entsteht beispielsweise durch Sulfatierung des Ricinusöles mit Hilfe von SO₃ (Soap, Cosmet, Chem. Specialties 48, Nr. 6, 44 [1972*]*)*.* Das Gemisch wird anschließend mit Natronlauge oder Ammoniak neutralisiert, die dabei entstehenden Seifen sind gegen hartes Wasser weitgehend unempfindlich, waschen haut- und faserschonend und sind auch als anionische Emulgatoren geeignet. Türkischrotöl ist in den zur Anwendung kommenden Verdünnungen gut hautverträglich.

Die Gehalt der Lipidphase wird vorteilhaft größer als 20 Gew.-% gewählt, bevorzugt zwischen 30 und 60 Gew.-%, insbesondere bevorzugt zwischen 35 und 50 Gew.-%, jeweils bezogen auf das Gesamtgewicht der Zubereitung. Es ist gegebenenfalls ferner vorteilhaft, wenngleich nicht zwingend, wenn die Lipidphase zu mehr als 50 Gew.-% - bezogen auf das Gesamtgewicht der Lipidphase - an unpolaren Lipiden (mit einer Polarität ≥ 30 mN/m) enthält.

Vorteilhaft ist mindestens eine der Phasen durch einen Farbstoff eingefärbt, z. B. um dem Verbraucher die Phasentrennung anzuzeigen. Die Farbstoffe können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Der Gehalt an einem oder mehreren Farbstoffen ist vorteilhaft ≤ 0,05 Gew.-%, bezogen auf das Gesamtgewicht der Zubereitung. Zur Einfärbung eignen sich insbesondere öllösliche Farbstoffe.

Polysorbate sind Polyoxyethylenderivate, die u. a. unter der Bezeichnung Tween bekannt sind. Vorteilhaft im Sinne der vorliegenden Erfindung sind Polysorbat 20 (Polyoxyethylen-(20)sorbitanmonolaurat, CAS-Nr. 9005-64-5), welches sich durch folgende Strukturformel (I) auszeichnet, worin w + x + y + z einem Durchschnittswert von 20 und n = 10 entspricht.

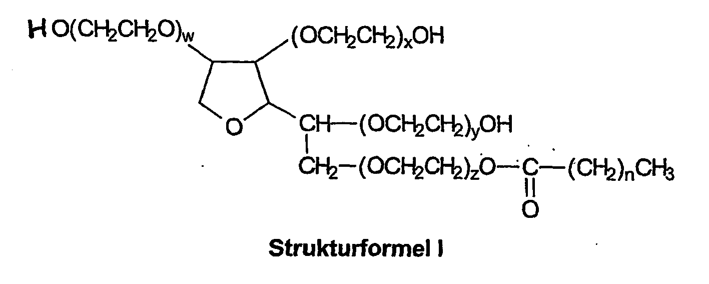

Polysorbat 21 (Polyoxyethylen(4)sorbitanmonolaurat, CAS-Nr. 9005-64-5) zeichnet sich ebenfalls durch Strukturformel (I) aus, worin w + x + y + z einem Durchschnittswert von 4 und n = 10 entspricht.

Polysorbat 40 (Polyoxyethylen(20)sorbitanmonopalmitat, CAS-Nr. 9005-66-7) zeichnet sich ebenfalls durch Strukturformel (I) aus, worin w + x + y + z einem Durchschnittswert von 20 und n = 14 entspricht.

Polysorbat 60 (Polyoxyethylen(20)sorbitanmonostearat, CAS-Nr. 9005-67-8) zeichnet sich ebenfalls durch Strukturformel (I) aus, worin w + x + y + z dem Durchschnittswert von 20 und n = 16 entspricht.

Polysorbat 61 (Polyoxyethylen(4)sorbitanmonostearat, CAS-Nr. 9005-67-8), zeichnet sich ebenfalls durch Strukturformel (I) aus, worin w + x + y + z einem Durchschnittswert von 4 und n = 16 entspricht.

Polysorbat 65 (Polyoxyethylen(20)sorbitantristearat, CAS-Nr. 9005-71-4) zeichnet sich durch Strukturformel (II) aus, worin w + x + y + z einem Durchschnittswert von 20 entspricht.

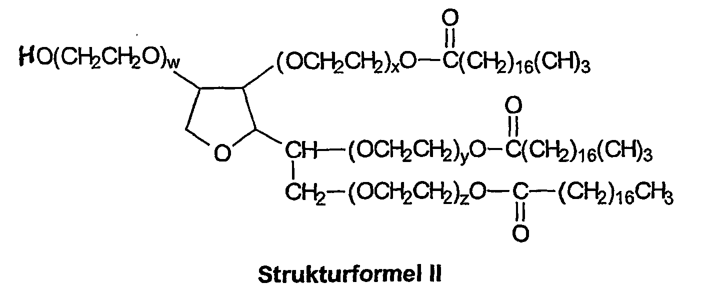

Polysorbat 80 (Polyoxyethylen(20)sorbitanmonooleat, CAS-Nr. 9005-65-6) zeichnet sich durch Strukturformel (III) aus, worin w + x + y + z einem Durchschnittswert von 20 entspricht.

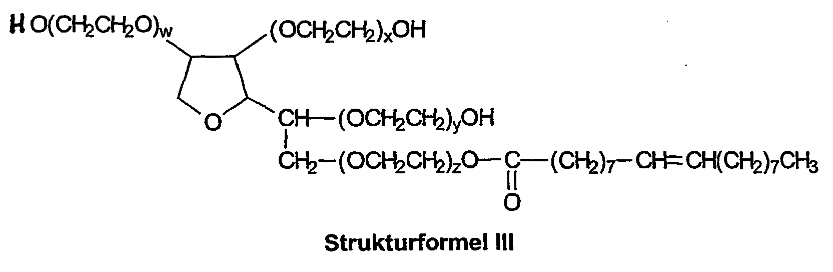

Polysorbat 81 (Polyoxyethylen(5)sorbitanmonooleat, CAS-Nr. 9005-65-6) zeichnet sich ebenfalls durch Strukturformel (III) aus, worin w + x + y + z einem Durchschnittswert von 5 entspricht.

Polysorbat 85 (Polyoxyethylen(20)sorbitantrioleat, CAS-Nr. 9005-70-3) zeichnet sich durch Strukturformel (IV) aus, worin w + x + y + z einem Durchschnittswert von 20 entspricht.

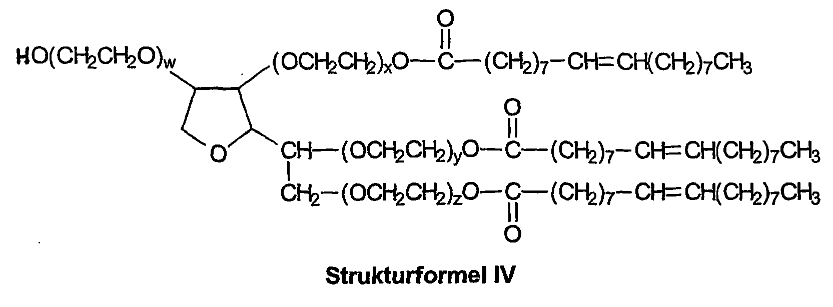

Besonders vorteilhaft im Sinne der vorliegenden Erfindung ist Polysorbat 20.
Die Menge an Polysorbaten (eine oder mehrere Verbindungen) in den Zubereitungen beträgt vorzugsweise 5 bis 20 Gew.%, besonders bevorzugt 10 bis 15Gew.-%, jeweils bezogen auf das Gesamtgewicht der Zubereitung.

Die erfindungsgemäßen Zusammensetzungen enthalten außer den vorgenannten Bestandteilen gegebenenfalls die in der Kosmetik üblichen Zusatzstoffe, beispielsweise Emulgatoren, Verdicker, Lösungsvermittler, Parfüm, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die erfindungsgemäßen Zubereitungen enthalten gegebenenfalls auch einen oder mehrere Emulgatoren. Vorteilhaft werden diese gewählt aus der Gruppe
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel R-COO-(-CH₂-CH₂-O-)ₙ -H, wobei R einen verzweigten oder unverzweigten Alkyl- oder Alkenylrest und n eine Zahl von 10 bis 40 darstellen,
- der veretherten Fettsäureethoxylate der allgemeinen Formel R-COO-(-CH₂-CH₂-O-)ₙ -R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙSO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen.
- der Fettalkoholpropoxylate der allgemeinen Formel R-O-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolether der allgemeinen Formel R-O-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel R-COO-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel R-COO-(-CH₂-CH(CH₃)-O-)ₙ-C(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel R-COO-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80,
- der propoxylierten Sorbitanester mit einem Propoxylierungsgrad von 3 bis 100,
- der Cholesterinpropoxylate mit einem Propoxylierungsgrad von 3 bis 100,
- der propoxylierten Triglyceride mit einem Propoxylierungsgrad von 3 bis 100,
- der Alkylethercarbonsäuren der allgemeinen Formel R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel R-O-(-CH₂-CH(CH₃)-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylatelpropoxylate der allgemeinen Formel R-O-Xₙ-Yₘ-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel R-COO-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel R-COO-Xₙ-Yₘ-H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Vorteilhaft ist insbesondere, Lösungsvermittler aus der Gruppe der Polyoxyethylen-Polyoxypropylen-Blockcopolymere zu wählen. Solche Blockcoploymere sind unter der Bezeichnung "Poloxamere" bekannt und zeichnen sich durch folgende Struktur aus:

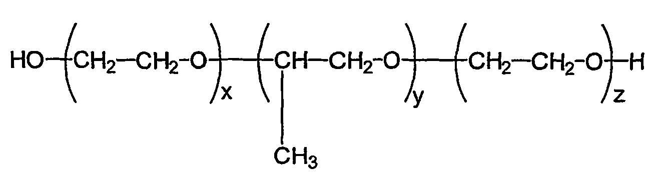

Dabei nimmt x vorteilhaft Werte zwischen 2 und 20 an. y nimmt vorteilhaft Werte zwischen 10 und 50 an. z nimmt vorteilhaft Werte zwischen 2 und 20 an.

Wenn Zubereitungen gemäß der vorliegenden Erfindung außer den erfindungsgemäßen Tensiden weitere Tenside enthalten sollen, so wird bevorzugt, deren Konzentration in bezug auf das Gewicht der Gesamtzusammensetzung nicht größer als 5 Gew.-% zu wählen.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Pentat-, Hexa-, Heptahioninsulfoximin) in sehr geringen verträglichen Dosierungen (z. B. pmol bis µmol/kg), ferner (Metall)-Chelatoren (z. B.α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. gamma-Linolensäure, Linolsäure, Ölsäure), Folsäure und deren Derivate, Ubiquinon und Ubiquinol deren Derivate, Vitamin C und Derivate (z. B. Ascorbylpalmitate, Mg - Ascorbylphosphate, Ascorbylacetate), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Zur Anwendung werden die kosmetischen und dermatologischen Zubereitungen in der für Kosmetika üblichen Weise auf die Haut und/oder die Haare in ausreichender Menge aufgebracht.

Durch Schütteln unmittelbar vor der Anwendung kann eine optisch homogene Mischung erzeugt werden, welche sich nach kurzer Zeit (d. h. je nach Formulierung in Sekunden, Minuten, Stunden) wieder trennt.

Vorteilhaft im Sinne der vorliegenden Erfindung sind insbesondere solche Formulierungen, welche sich durch intensives Schütteln verdicken. Durch die derartige Viskositätserhöhung der Formulierungen wird beispielsweise die Trennungsgeschwindigkeit der Phasen herabgesetzt.

Die erfindungsgemäßen Zubereitungen können vorteilhaft als Wannen(bad)zubereitungen, also beispielsweise als Ölbäder und dergleichen, aber auch als Duschpräparate - wie z. B. als Duschöle - oder als Haarwaschmittel verwendet werden.

Die nachfolgenden Beispiele sollen die vorliegende Erfindung verdeutlichen. Die Zahlenwerte in den Beispielen bedeuten Gewichtsprozente, bezogen auf das Gesamtgewicht der jeweiligen Zubereitungen.

### Beispiele;

### 2-Phasen-Ölbäder

| | **1** | **2** | **3** | **4** | **5** |
|---|---|---|---|---|---|
| Triisopropanolammoniumfettalkohol-ethersulfat (TIPA-Laurethsulfat) | 24% | 26% | 24% | 26% | 30% |
| 1,2-Propylenglykol | 1,8% | 1,8% | 1,8% | 1,8% | 1,8% |
| Polyoxyethylen(20)sorbitanmonolaurat | 13% | 13% | 13% | 13% | 13% |
| Türkischrotöl 100% | 2,4% | 2,4% | 2,4% | -- | 2,4% |
| Glycerin | 12% | 12% | 12% | 12% | 10% |
| Parfüm | 2% | 2% | 2% | 2% | 2% |
| Farbstoff | 0,002% | 0,002% | 0,002% | 0,002% | 0,002% |
| Rizinusöl | 3% | --- | | 4% | --- |
| Kokosfettsärediethanolamid | --- | --- | 2% | --- | --- |
| Paraffinöl | ad 100 | ad 100 | ad 100 | ad 100 | ad 100 |

## Patentansprüche

1. Kosmetische oder dermatologische Reinigungszubereitungen, welche in mindestens zwei Phasen vorliegen, enthaltend
(a) mindestens ein waschaktives Tensid, gewählt aus der Gruppe der Trialkyl- und/oder Trialkanolaminsalze der Fettalkoholsulfate und Fettalkoholethersulfate,
(b) einen Gehalt von 20 bis 70 Gew.-%, bezogen auf das Gesamtgewicht der Zubereitungen, einer oder mehrerer Ölkomponenten, gewählt aus der Gruppe der unpolaren Lipide mit einer Polarität ≥ 30 mN/m und
(c) mindestens einem Polysorbat.
wobei die Reinigungszubereitung wasserfrei ist.

2. Reinigungszubereitungen nach Anspruch 1, **dadurch gekennzeichnet, dass** sie ferner Glycerin enthalten.

3. Reinigungszubereitungen nach Anspruch 2, **dadurch gekennzeichnet, dass** das Glycerin als "chemisch rein" oder "reinst" nach DAB zu bezeichnen ist.

4. Reinigungszubereitungen nach Anspruch 1, **dadurch gekennzeichnet, dass** die Ölkomponenten gewählt werden aus der Gruppe der Mineralöle und/oder der Paraffinöle.

5. Reinigungszubereitungen nach Anspruch 1, **dadurch gekennzeichnet, dass** die Fettalkoholsulfate bzw. Fettalkoholethersulfate folgende Struktur aufweisen: 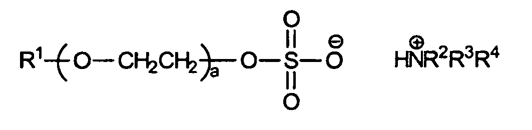 wobei a Werte von 0 bis 10, vorteilhaft 1 bis 5, annimmt und R¹ gewählt wird aus der Gruppe der verzweigten und unverzweigten Alkylgruppen mit 6 bis 24 Kohlenstoffatomen und R², R³ und R⁴ unabhängig voneinander gewählt werden aus der Gruppe der verzweigten und unverzweigten Alkyl- und Hydroxyalkylreste mit 1 bis 24 Kohlenstoffatomen.

6. Reinigungszubereitungen nach Anspruch 5, **dadurch gekennzeichnet, dass** R², R³ und R⁴ folgende Struktur aufweisen:

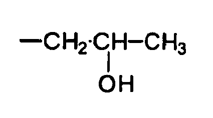

7. Reinigungszubereitungen nach einem der vorhergehenden Ansprüche, **dadurch gekennzeichnet, dass** als waschaktives Tensid TIPA-Laurethsulfat gewählt wird.

8. Reinigungszubereitungen nach einem der vorhergehenden Ansprüche, **dadurch gekennzeichnet, dass** als Polysorbat Polyoxyethylen(20)sorbitanmonolaurat gewählt wird.

9. Reinigungszubereitungen nach einem der vorhergehenden Ansprüche, **dadurch gekennzeichnet, dass** sie ferner ein oder mehrere Türkischrotöle enthalten.

10. Reinigungszubereitungen nach einem der vorhergehenden Ansprüche, **dadurch gekennzeichnet, dass** sie ferner einen oder mehrere Farbstoffe enthalten.

## Claims

1. Cosmetic or dermatological cleansing preparations which are present in at least two phases, comprising
a) at least one washing-active surfactant selected from the group of trialkyl- and/or trialkanolamine salts of fatty alcohol sulphates and fatty alcohol ether sulphates,
b) a content of from 20 to 70% by weight, based on the total weight of the preparations, of one or more oil components selected from the group of nonpolar lipids having a polarity ≥ 30 mN/m and
c) at least one polysorbate
where the cleansing preparation is water-free.

2. Cleansing preparations according to Claim 1, **characterized in that** they further comprise glycerol.

3. Cleansing preparations according to Claim 2, **characterized in that** the glycerol is to be denoted "chemically pure" or "highest-purity" according to German Pharmacopoeia.

4. Cleansing preparations according to Claim 1, **characterized in that** the oil components are selected from the group of mineral oils and/or paraffin oils.

5. Cleansing preparations according to Claim 1, **characterized in that** the fatty alcohol sulphates or fatty alcohol ether sulphates have the following structure: 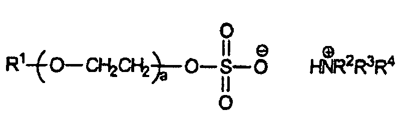 where a assumes values from 0 to 10, advantageously 1 to 5, and R¹ is selected from the group of branched and unbranched alkyl groups having 6 to 24 carbon atoms, and R², R³ and R⁴, independently of one another, are selected from the group of branched and unbranched alkyl- and hydroxyalkyl radicals having 1 to 24 carbon atoms.

6. Cleansing preparations according to Claim 5, **characterized in that** R², R³ and R⁴ have the following structure:

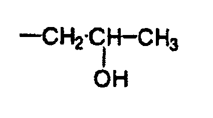

7. Cleansing preparations according to one of the preceding claims, **characterized in that** the washing-active surfactant selected is TIPA laureth sulphate.

8. Cleansing preparations according to one of the preceding claims, **characterized in that** the polysorbate selected is polyoxyethylene(20) sorbitan monolaurate.

9. Cleansing preparations according to one of the preceding claims, **characterized in that** they also comprise one or more Turkey red oils.

10. Cleansing preparations according to one of the preceding claims, **characterized in that** they also comprise one or more dyes.

## Revendications

1. Compositions cosmétiques ou dermatologiques de nettoyage, dans lesquelles existent au moins deux phases, contenant
a) au moins un agent tensioactif actif en lavage, choisi dans le groupe des sels de trialkylamine et/ou de trialcanolamine des sulfates d'alcools gras et des éthersulfates d'alcools gras,
b) une teneur de 20 à 70% en poids, par rapport au poids total des compositions, en un ou plusieurs composants huileux choisis dans le groupe des lipides non polaires présentant une polarité ≥ 30 mN/m et
c) au moins un polysorbate
la composition de nettoyage étant exempte d'eau.

2. Compositions de nettoyage selon la revendication 1, **caractérisées en ce qu'**elles contiennent en outre du glycérol.

3. Compositions de nettoyage selon la revendication 2, **caractérisées en ce que** le glycérol est un glycérol appelé "chimiquement pur" ou "pur" selon DAB.

4. Compositions de nettoyage selon la revendication 1, **caractérisées en ce que** les composants huileux doivent être choisis dans le groupe des huiles minérales et/ou des huiles de paraffine.

5. Compositions de nettoyage selon la revendication 1, **caractérisées en ce que** les sulfates d'alcools gras ou les éthersulfates d'alcools gras présentent la structure suivante : 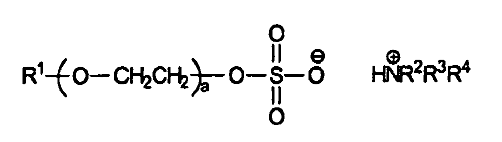 où a présente des valeurs de 0 à 10, avantageusement de 1 à 5, et R¹ est choisi dans le groupe formé par les groupes alkyle ramifiés et non ramifiés comprenant 6 à 24 atomes de carbone
et R², R³ et R⁴ sont choisis indépendamment l'un de l'autre du groupe des radicaux alkyle et hydroxyalkyle ramifiés et non ramifiés comprenant 1 à 24 atomes de carbone.

6. Compositions de nettoyage selon la revendication 5, **caractérisées en ce que** R², R³ et R⁴ présentent la structure suivante :

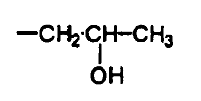

7. Compositions de nettoyage selon l'une quelconque des revendications précédentes, **caractérisées en ce qu'**on choisit du TI-PA-laurethsulfate comme agent tensioactif actif en lavage.

8. Compositions de nettoyage selon l'une quelconque des revendications précédentes, **caractérisées en ce qu'**on choisit du monolaurate de polyoxyéthylène(20)sorbitane comme polysorbate.

9. Compositions de nettoyage selon l'une quelconque des revendications précédentes, **caractérisées en ce qu'**elles contiennent en outre une ou plusieurs huiles de ricin sulfonées (huile rouge de Turquie).

10. Compositions de nettoyage selon l'une quelconque des revendications précédentes, **caractérisées en ce qu'**elles contiennent en outre un ou plusieurs colorants.
